# EUROPEAN PATENT SPECIFICATION

(11) **EP 1 392 316 B1**
(45) Date of publication and mention of the grant of the patent: **20.04.2005**
(21) Application number: 02750872.0
(22) Date of filing: 02.05.2002
(51) Int. Cl.: A61K 31/55, C07D 403/12, A61P 25/00

(54) **BENZO[D]AZEPINE DERIVATIVES AS 5-HT6 RECEPTOR ANTAGONISTS.**
BENZO[D]AZEPIN-DERIVATIVE ALS 5-HT6-REZEPTOR-ANTAGONISTEN
DERIVES DE BENZO[D]AZEPINE UTILES COMME ANTAGONISTES DU RECEPTEUR 5-HT6

(30) Priority: 08.05.2001 GB 0111186
(43) Date of publication of application: 03.03.2004
(73) Proprietor: SMITHKLINE BEECHAM PLC, Brentford, Middlesex TW8 9GS (GB)
(72) Inventor: BROMIDGE, Steven Mark, GlaxoSmithKline, Harlow, Essex CM19 5AW (GB); MOSS, Stephen Frederick, GlaxoSmithKline, Harlow, Essex CM19 5AW (GB)
(74) Representative: Billson, Siân Catherine
(86) International application number: PCT/EP2002/004804
(87) International publication number: WO 2002/089811

(56) References cited:
- WO-A-00/00203
- WO-A-98/27081
- MARYANOFF B E ET AL: "Azepinoindole derivatives with high affinity for brain dopamine and serotonin receptors" BIOORGANIC & MEDICINAL CHEMISTRY LETTERS, OXFORD, GB, vol. 8, no. 8, 21 April 1998 (1998-04-21), pages 983-988, XP004137004 ISSN: 0960-894X

## Description

This invention relates to novel benzazepine compounds having pharmacological activity, processes for their preparation, to compositions containing them and to their use in the treatment of CNS and other disorders.

WO 98/27081 discloses a series of aryl sulphonamide compounds that are said to be 5-HT₆ receptor antagonists and which are claimed to be useful in the treatment of various CNS disorders. WO 99/47516 and WO 99/65906 both disclose a series of indole derivatives that are claimed to possess 5-HT₆ receptor affinity.

A structurally novel class of compounds has now been found which also possess 5-HT₆ receptor affinity. The present invention therefore provides, in a first aspect, a compound of formula (I) or a pharmaceutically acceptable salt thereof: 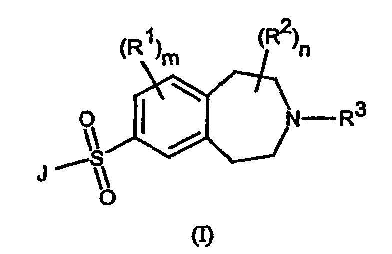 wherein
R¹ is halogen, C₁₋₆alkyl, C₁₋₆alkoxy, C₁₋₆alkanoyl, CN, CF₃ or OCF₃;
R² is C₁₋₆alkyl;
R³ is hydrogen or a C₁₋₁₀alkyl group optionally substituted by one, two or three substituents selected from the group consisting of halogen, C₁₋₆alkoxy, CN, amino, mono- or
di-C₁₋₆alkylamino or a group -C(O)OR⁷ where R⁷ is hydrogen or C₁₋₆ alkyl; m is 0 - 3;
n is 0 - 8;
J is selected from a group of formula (a), (b) or (c) in which
(a) is a group 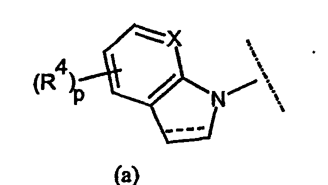 wherein R⁴ is halogen, C₁₋₆alkyl, C₁₋₆alkoxy, C₁₋₆alkanoyl, C₁₋₆alkylthio, hydroxy, CN, CF₃, NO₂, OCF₃, phenyl optionally substituted by groups as defined for R¹ above, benzyl, phenyloxy, benzyloxy or C₃₋₆cycloalkyloxy or a group (CH₂)_{q} NR⁸R⁹ where q is 0, 1 or 2 and R⁸ and R⁹ are independently hydrogen or C₁₋₆alkyl;
   p is 0, 1, 2, 3 or 4;
   X is CH or N; and
   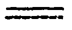 is a single or double bond;
(b) is a group 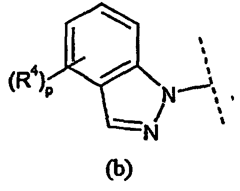 in which R⁴ and p are as defined for group (a) above; and
(c) is a group 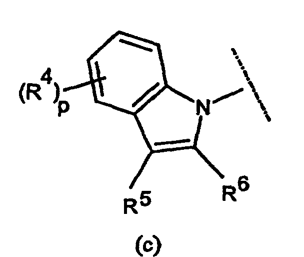
in which R⁴ and p are as defined for group (a) above and R⁵ and R⁶ combine together to form a 5- to 7- membered carbocyclic or heterocyclic ring optionally substituted by groups as defined for R¹ above.

Alkyl groups, whether alone or as part of another group, may be straight chain or branched. The term 'halogen' is used herein to describe, unless otherwise stated, a group selected from fluorine, chlorine, bromine or iodine.

When present (i.e. when m is other than 0), the group(s) R¹ may be substituted at any suitable carbon atom within the benzene ring. When m is 2 or 3 the groups R¹ may be the same or different. Preferred R¹ groups are halogen (particularly fluorine, chlorine or bromine) or a C₁₋₆alkyl group (particularly methyl). Preferably m is 0 or 1, most preferably 0.

When present (i.e. when n is other than 0), the group(s) R² may be substituted at any suitable carbon atom within the azepine ring. When n is two or more the groups R² may be same or different. A particularly preferred R² group is methyl. Preferably, n is 0, 1 or 2.
Preferably R³ is hydrogen, methyl or ethyl, most preferably hydrogen.

### Within the definition of J formula (a)

The groups R⁴ can be substituted at any suitable carbon atom within the indole ring (i.e. when 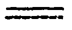 is a double bond) or indoline (i.e. when 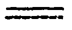 is a single bond) ring. When p is other than 0, R⁴ is preferably halogen (particularly fluorine, chlorine or bromine), a C₁₋₆alkyl group (particularly methyl), a C₁₋₆alkoxy group (particularly methoxy), benzyl, optionally substituted phenyl or a group (CH₂)_{q}NR⁸R⁹ (particularly CH₂NMe₂). When p is 2, 3 or 4 the groups R⁴ may be the same or different. Preferably, 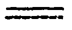 is a double bond.

### Within the definition of J formula (b)

The groups R⁴ can be substituted at any suitable carbon atom in the indazole ring. Preferred R⁴ groups include those given for formula (a) above.

### Within the definition of J formula (c)

The groups R⁴ can be substituted at any suitable carbon atom. Preferred R⁴ groups include those given for formula (a) above.
The 5- to 7- membered heterocyclic ring formed by the combination of groups R⁵ and R⁶ may be saturated, unsaturated or partially saturated. Preferably R⁵ and R⁶ combine together to form a 6 membered carbocyclic or heterocyclic ring. Most preferably, R⁵ and R⁶ combine such that the group J is a carbazole or tetrahydrocarbazole ring.

Particularly preferred compounds according to the invention include examples 1 - 33 (as shown below) or a pharmaceutically acceptable salt thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid.

The compounds of formula (I) may be prepared in crystalline or non-crystalline form, and, if crystalline, may optionally be hydrated or solvated. This invention includes within its scope stoichiometric hydrates as well as compounds containing variable amounts of water.

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

In a further aspect, the present invention also provides a process for the preparation of a compound of formula (1) or a pharmaceutically acceptable salt thereof, which process comprises the coupling of a compound of formula (II):

J-H (II)

in which J is as defined in formula (I) with a compound of formula (III) or a protected derivative thereof; 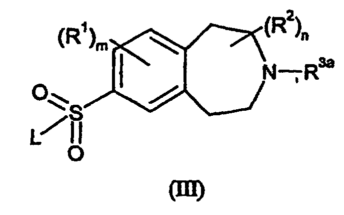 in which R¹, R², m and n are as defined in formula (1), R^{3a} is an N protecting group or R³ and L is a leaving group and optionally thereafter:
- converting a compound of formula (1) into another compound of formula (1);
- removing any protecting groups;
- forming a pharmaceutically acceptable salt.

Suitable leaving groups include halogen, in particular chloro. The reaction of compounds of formulae (II) and (III) may be carried out by mixing the two reagents together, optionally under phase-transfer conditions, in a mixture of an inert organic solvent such as tetrahydrofuran with an aqueous base such as sodium hydroxide with the addition of a suitable phase-transfer catalyst such as tetrabutylammonium hydroxide. Alternatively, the reaction of compounds of formulae (II) and (III) may be carried out by treating a compound of formula (II) with a suitable base such as sodium hydride or sodium hexamethyldisilazane (NaHMDS) in an inert solvent such as tetrahydofuran or N,N-dimethylformamide to form the anion of (II) and then treating this with a compound of formula (III) in an inert solvent. For compounds of formula (1) in which J is a group of formula (a) and 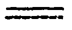 is a single bond, the reaction of compounds of formulae (II) and (III) is carried out by mixing the two reagents together, optionally in an inert solvent such as dichloromethane with or without the addition of a suitable base such as triethylamine or pyridine.

Compounds of formula (I) can be converted into further compounds of formula (I) using standard techniques. The following example is given by way of illustration. For compounds of formula (I) wherein R³ is hydrogen, it is possible to introduce an alternative R³ group by conventional alkylation using 1 molar equivalent of an alkylhalide and 1 molar equivalent of a suitable base in an inert solvent.

It will be appreciated by those skilled in the art that it may be necessary to protect certain reactive substituents during some of the above procedures. Standard protection and deprotection techniques, such as those described in Greene T.W. Protective groups in organic synthesis', New York, Wiley (1981), can be used. For example, primary amines can be protected as phthalimide, benzyl, benzyloxycarbonyl or trityl derivatives. Carboxylic acid groups can be protected as esters. Aldehyde or ketone groups can be protected as acetals, ketals, thioacetals or thioketals. Deprotection of such groups is achieved using conventional procedures well known in the art.

Compounds of formulae (II) and (III) are commercially available, may be prepared using procedures described herein or by analogous methods thererto or according to known methods.

Pharmaceutically acceptable salts may be prepared conventionally by reaction with the appropriate acid or acid derivative.

Compounds of formula (I) and their pharmaceutically acceptable salts have 5-HT₆ receptor activity and are believed to be of potential use in the treatment of certain CNS disorders such as anxiety, depression, epilepsy, obsessive compulsive disorders, migraine, cognitive memory disorders e.g. Alzheimers disease and age related cognitive decline, Parkinsons Disease, ADHD (Attention Deficit Disorder/Hyperactivity Syndrome), sleep disorders (including disturbances of Circadian rhythym), feeding disorders such as anorexia and bulimia, panic attacks, withdrawal from drug abuse such as cocaine, ethanol, nicotine and benzodiazepines, schizophrenia, and also disorders associated with spinal trauma and/or head injury such as hydrocephalus. Compounds of the invention are also expected to be of use in the treatment of certain GI (gastrointestinal) disorders such as IBS (Irritable Bowel Syndrome). Compounds ofthe invention are further expected to be of use in the treatment of obesity and mild cognitive impairment.

Thus the invention also provides a compound of formula (I) or a pharmaceutically acceptable salt thereof, for use as a therapeutic substance, in particular in the treatment or prophylaxis of the above disorders. In particular the invention provides for a compound of formula (I) or a pharmaceutically acceptable salt thereof, for use in the treatment of depression, anxiety, Alzheimers disease, age related cognitive decline and ADHD. Also in particular the invention provides for a compound of formula (I) or a pharmaceutically acceptable salt thereof, for use in the treatment of obesity, mild cognitive impairment and schizophrenia.

The invention further provides a method of treatment or prophylaxis of the above disorders, in mammals including humans, which comprises administering to the sufferer a therapeutically effective amount of a compound of formula (I) or a pharmaceutically acceptable salt thereof.

In another aspect, the invention provides the use of a compound of formula (I) or a pharmaceutically acceptable salt thereof in the manufacture of a medicament for use in the treatment or prophylaxis of the above disorders.

In order to use the compounds of formula (I) in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier or excipient

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 20.0 mg, for example 0.2 to 5 mg; and such unit doses may be administered more than once a day, for example two or three times a day, so that the total daily dosage is in the range of about 0.5 to 100 mg; and such therapy may extend for a number of weeks or months.

All publications, including but not limited to patents and patent applications, cited in this specification are herein incorporated by reference as if each individual publication were specifically and individually indicated to be incorporated by reference herein as though fully set forth.

The following Descriptions and Examples illustrate the preparation of compounds of the invention.

### Description 1

### 1-[7-(Indole-1-sulfonyl)-1,2,4,5-tetrahydro-benzo[d]azepin-3-yl]-ethanone (D1)

To a vigorously stirred solution of indole (31 mg, 0.26 mmol) in tetrahydrofuran (1mL) at room temperature, was added a 40% aqueous solution of tetrabutylammonium hydroxide (14 uL, 14.7 umol) followed by a 50% aqueous solution of sodium hydroxide (1 mL). After stirring the mixture for 10 minutes, a solution of 3-acetyl-2,3,4,5-tetrahydro-1H-benzo[d]azepine-7-sulfonyl chloride(for synthesis see patent: DE 2027436 (1970); Chem. Abstr. 1972, 76: 99535) (75 mg, 0.26 mmol) in tetrahydrofuran (1 mL) was added. A further quantity of the sulfonyl chloride (35 mg, 0.12 mmol) in tetrahydrofuran (1 mL) was added after 1 hour and vigorous stirring was continued for a further 2 hours. To the reaction mixture was then added water (3 mL) and ethyl acetate (3 mL). After shaking the mixture, the layers were separated and the organic phase was washed with brine (3 mL), dried (MgSO₄) and filtered. The filtrate was concentrated *in vacuo* to an oil which was purified by column chromatography over silica gel, eluting with a gradient of dichloromethane/methanol, to afford the title compound (D1) (79 mg, 82%), δH (CDCl₃)/ppm (rotamers) 2.21, 2.15 (3H, 2 x s), 2.89-2.94 (4H, m), 3.51-3.55 (2H, m), 3.65-3.68 (2H, m), 6.66 (1H, d, J = 4.0Hz), 7.19-7.32 (3H, m), 7.53-7.68 (4H, m), 7.98 (1H, d, J = 8.0Hz). MS: m/z (MH⁺) 369.

### Description 2

### 1-[7-(2,3-Dihydro-indole-1-sulfonyl)-1,2,4,5-tetrahydro-benzo[d]azepin-3-yl]-ethanone (D2)

A solution of 2,3-dihydro-1*H*-indole (42mg, 0.35mmol) in dichloromethane (1ml) was added to a stirred solution of 3-acetyl-2,3,4,5-tetrahydro-1*H*-benzo[d]azepine-7-sulfonyl chloride (100mg, 0.35mmol) in dichloromethane (1ml) at room temperature under argon. *N,N*-diisopropylethylamine (61µl, 0.35mmol) was then added and the solution stirred for a further 18h. The reaction mixture was diluted with dichloromethane (10ml) and the solution was washed with brine (10ml), dried (MgSO₄) and concentrated in vacuo to an oil. The oil was purified by column chromatography over silica gel eluting with a gradient of dichloromethane/methanol to afford the title compound (D2) as an oil (114mg, 88%). MS: m/z (MH⁺) 371.

### Description 3

### 1-[7-(5-Bromo-2,3-dihydro-indole-1-sulfonyl)-1,2,4,5-tetrahydro-benzo[d]azepin-3-yl]-ethanone (D3)

The title compound (D3) was prepared as described in Description 2 by reacting 5-bromo-2,3-dihydro-1*H*-indole with 3-acetyl-2,3,4,5-tetrahydro-1H-benzo[d]azepine-7-sulfonyl chloride. Yield = 73%. MS: m/z (MH⁺) 449/451.

### Example 1

### 7-(Indole-1-sulfonyl)-2,3,4,5-tetrahydro-1-H-benzo[d]azepine (E1)

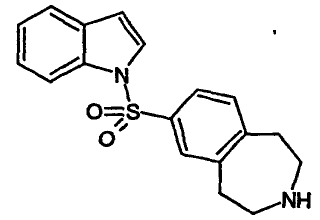

A stirred solution of 1-[7-(indole-1-sulfonyl)-1,2,4,5-tetrahydro-benzo[d]azepin-3-yl]-ethanone (D1) (73 mg, 0.2 mmol) in n-butanol (1.5 mL) and 3M hydrochloric acid (3 mL) was heated at reflux for 7 hours. To the cooled mixture was added ethyl acetate (20 mL) and a saturated aqueous solution of sodium hydrogen carbonate (20 mL) and the whole was shaken. After separating the layers, the organic phase was washed with brine (10 mL), dried (MgSO₄) and filtered. The filtrate was concentrated *in vacuo* to an oil which was purified by column chromatography over silica gel, eluting with a gradient of dichloromethane/methanol, to afford the title compound (E1) (36 mg). Treatment of a solution of this material in dichloromethane/diethyl ether (3 mL) with oxalic acid (1.5 equivalents) afforded the corresponding crystalline oxalate salt (39 mg, 47%), δH (CD₃OD)/ppm 3.29-3.38 (8H, m), 6.88 (1H, d, J = 3.6Hz), 7.38 (1H, t, J = 7.6Hz), 7.45 (1H, t, J = 7.6Hz), 7.51 (1H, d, J = 8.0Hz), 7.68 (1H, d, J = 6.8Hz), 7.80 (1H, d, J = 3.6Hz), 7.92-8.12 (2H, m), 8.12 (1H, d, J = 7.6Hz). MS: m/z (MH⁺) 327.

The following compounds were prepared as oxalate salts by a sequential two step procedure as described in Description D1, treating the appropriate indole or indazole with 3-acetyl-2,3,4,5-tetrahydro-1*H*-benzo[d]azepine-7-sulfonyl chloride, and Example E1, in which N-deacetylation is carried out.

| **Compound** | **MH**^{**+**} |
|---|---|
| **7-(4-Chloro-indole-1-sulfonyl))-2,3,4,5-tetrahydro-1-*H*-benzo[d] azepine (E2)** | 361/363 |
| **7-(6-Chloro-indole-1-sulfonyl)-2,3,4,5-tetrahydro-1-*H*-benzo[d] azepine (E3)** | 361/363 |
| **7-(3-Methyl-indole-1-sulfonyl)-2,3,4,5-tetrahydro-1*-H*-benzo[d] azepine (E4)** | 341 |
| **7-[2-(4-Fluoro-phenyl)-indole-1-sulfonyl]-2,3,4,5-tetrahydro-1*-H*-benzo[d] azepine (E5)** | 421 |
| **7-(2-Methyl-indole-1-sulfonyl)-2,3,4,5-tetrahydro-1-*H*-benzo[d] azepine (E6)** | 341 |
| **Dimethyl-[1-(2,3,4,5-tetrahydro-1*H*-benzo[d]azepine-7-sulfonyl)-1*H*-indol- 3-ylmethyl]-amine (E7)** | 384 |
| **7-(3-Phenyl-indole-1-sulfonyl)-2,3,4,5-tetrahydro-1-*H*-benzo[d] azepine (E8)** | 403 |
| **7-(3-Benzyl-indole-1-sulfonyl)-2,3,4,5-tetrahydro-1-*H*-benzo[d] azepine (E9)** | 417 |
| **7-(7-Bromo-indole-1-sulfonyl)-2,3,4,5-tetrahydro-1-*H*-benzo[d] azepine (E10)** | 405/407 |
| **7-(4-Methoxy-indole-1-sulfonyl)-2,3,4,5-tetrahydro-1-*H*-benzo[d] azepine (E11)** | 357 |
| **7-(4-Methyl-indole-1-sulfonyl)-2,3,4,5-tetrahydro-1-*H*-benzo[d] azepine (E12)** | 341 |
| **7-(5-Bromo-indole-1-sulfonyl)-2,3,4,5-tetrahydro-1-*H*-benzo[d] azepine (E13)** | 405/407 |
| **7-(5-Fluoro-indole-1-sulfonyl)-2,3,4,5-tetrahydro-1-*H*-benzo[d] azepine (E14)** | 345 |
| **7-(6-Methoxy-indole-1-sulfonyl)-2,3,4,5-tetrahydro-1-*H*-benzo[d] azepine (E15)** | 357 |
| **7-(5-Chloro-2-methyl-indole-1-sulfonyl)-2,3,4,5-tetrahydro-1-*H*-benzo[d] azepine (E16)** | 375/377 |
| **7-(6-Fluoro-indole-1-sulfonyl)-2,3,4,5-tetrahydro-1-*H*-benzo[d] azepine (E17)** | 345 |
| **7-(7-Chloro-indole-1-sulfonyl)-2,3,4,5-tetrahydro-1*-H*-benzo[d] azepine (E18)** | 361/363 |
| **9-(2,3,4,5-Tetrahydro-1*H*-benzo[d]azepine-7-sulfonyl)-2,3,4,9-tetrahydro- 1H-carbazole (E19)** | 381 |
| **7-(4,5,6,7-Tetrafluoro-indole-1-sulfonyl)-2,3,4,5-tetrahydro-1*-H*-benzo[d]azepine (E20)** | 399 |
| **7-(2,3-Dimethyl-indole-1-sulfonyl)-2,3,4,5-tetrahydro-1*-H*-benzo[d] azepine (E21)** | 355 |
| **7-(3-Chloro-indazole-1-sulfonyl)-2,3,4,5-tetrahydro-1-*H*-benzo[d] azepine (E22)** | 362/364 |
| **7-(5,6-Dichloro-indole-1-sulfonyl)-2,3,4,5-tetrahydro-1-*H*-benzo[d] azepine (E23)** | 395/397 |
| **7-(7-Nitro-indole-1-sulfonyl)-2,3,4,5-tetrahydro-1*H*-benzo[d]azepine (E24)** | 372 |
| **9-(2,3,4,5-Tetrahydro-1*H*-benzo[d]azepine-7-sulfonyl)-9*H*-carbazole (E25)** | 377 |
| **7-(4,6-Difluoro-indole-1-sulfonyl)-2,3,4,5-tetrahydro-1*H*-benzo[d]azepine (E26)** | 363 |
| **7-(6-Trifluoromethyl-indole-1-sulfonyl)-2,3,4,5-tetrahydro-1*H*-benzo(d]azepine (E27)** | 395 |
| **7-(5,6-Difluoro-indole-1-sulfonyl)-2,3,4,5-tetrahydro-1*H*-benzo[d]azepine (E28)** | 363 |
| **Dimethyl-[1-(2,3,4,5-tetrahydro-1*H*-benzo[d]azepine-7-sulfonyl)-1*H*-indol- 3-ylmethyl]-amine (E29)** | 384 |

### Example 30

### 7-(Indole-1-sulfonyl)-3-methyl-2,3,4,5-tetrahydro-1-H-benzo[d]azepine (E30)

Triethylamine (0.38m1, 2.8mmol) and sodium hydride (60% weight dispersion in mineral oil, 17mg, 0.4mmol) were added to a solution of 7-(indole-1-sulfonyl)-2,3,4,5-tetrahydro-1 *H*-benzo[d]azepine (E1) (100mg, 0.28mmol) in 1,2-dimethoxyethane (5ml) under argon at 0°C, and the reaction mixture was stirred for 30 mins. Iodomethane (26µl, 0.4mmol) was added and the reaction mixture allowed to warm to room temperature and stirred overnight. After addition of saturated NH₄Cl solution and basification with 5% NaOH, the whole was extracted with dichloromethane. The organic layer was washed with brine (5ml), dried (MgSO₄) and filtered. The filtrate was concentrated *in vacuo* to a solid which was purified by column chromatography over silica gel, eluting with a gradient of dichloromethane/methanol, to afford the title compound (E30) (37 mg, 39%) as a colourless oil. This was dissolved in dichloromethane (1ml) and stirred with 1M HCl in ether (11µl, lequiv.) to afford the HCl salt of the title compound (E30) (32mg, 30%), δH (CD₃OD)/ppm 2.34 (3H, s), 2.50-2.52 (4H, m), 2.88-2.93 (4H, m), 6.65 (1H, d, J=3.7Hz), 7.11-7.26 (3H, m), 7.51-7.61 (4H, m), 7.97 (1H, d). MS: m/z (MH⁺) 341.

### Example 31

### 3-Ethyl-7-(indole-1-sulfonyl)-2,3,4,5-tetrahydro-1-H-benzo[d]azepine (E31)

The hydrochloride salt of the title compound (E31) was prepared by the same procedure as described in Example 30, treating 7-(indole-1-sulfonyl)-2,3,4,5-tetrahydro-1-*H*-benzo[d]azepine (E1) with iodoethane. Yield = 54%. MS: m/z (MH⁺) 355.

### Example 32

### 7-(2,3-Dihydro-indole-1-sulfonyl)-2,3,4,5-tetrahydro-1H-benzo[d]azepine (E32)

The oxalate salt of the title compound (E32) was prepared from 1-[7-(2,3-dihydro-indole-1-sulfonyl)-1,2,4,5-tetrahydro-benzo[d]azepin-3-yl]-ethanone (D2) as described in Example 1. Yield = 22%. MS: m/z (MH⁺) 329.

### Example 33

### 7-(5-Bromo-2,3-dihydro-indole-1-sulfonyl)-2,3,4,5-tetrahydro-1H-benzo[d]azepine (E33)

The oxalate salt of the title compound (E33) was prepared from 1-[7-(5-bromo-2,3-dihydro-indole-1-sulfonyl)-1,2,4,5-tetrahydro-benzo[d]azepin-3-yl]-ethanone (D3) as described in Example 1. Yield = 23%. MS: m/z (MH⁺) 407/409.

### Pharmacological data

Compounds can be tested for 5-HT₆ receptor affinity according to the procedures outlined in WO 98/27081.

All examples were found to have a pKi in the range 7.7 - 9.7 at human cloned 5-HT₆ receptors.

## Claims

1. A compound of formula (I) or a pharmaceutically acceptable salt thereof: 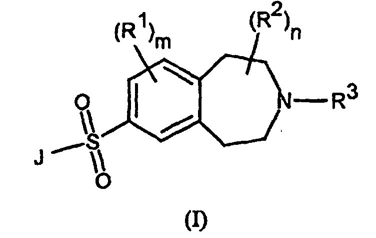 wherein
R¹ is halogen, C₁₋₆alkyl, C₁₋₆alkoxy, C₁₋₆alkanoyl, CN, CF₃ or OCF₃;
R² is C₁₋₆alkyl;
R³ is hydrogen or a C₁₋₁₀alkyl group optionally substituted by one, two or three substituents selected from the group consisting of halogen, C₁₋₆alkoxy, CN, amino, mono- or
di-C₁₋₆alkylamino or a group -C(O)OR⁷ where R⁷ is hydrogen or C₁₋₆ alkyl; m is 0 - 3;
n is 0 - 8;
J is selected from a group of formula (a), (b) or (c) in which
(a) is a group 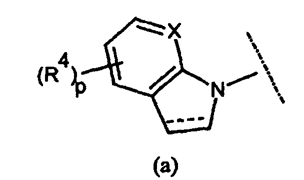 wherein R⁴ is halogen, C₁₋₆alkyl, C₁₋₆alkoxy, C₁₋₆alkanoyl, C₁₋₆alkylthio, hydroxy, CN, CF₃, NO₂, OCF₃, phenyl optionally substituted by groups as defined for R¹ above, benzyl, phenyloxy, benzyloxy or C₃₋₆cycloalkyloxy or a group (CH₂)_{q} NR⁸R⁹ where q is 0, 1 or 2 and R⁸ and R⁹ are independently hydrogen or C₁₋₆alkyl;
p is 0, 1, 2, 3 or 4;
X is CH or N; and
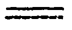 is a single or double bond;
(b) is a group 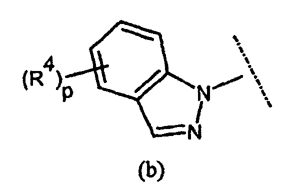 in which R⁴ and p are as defined for group (a) above; and
(c) is a group 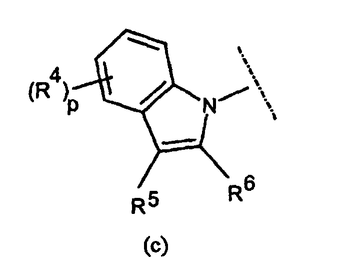
in which R⁴ and p are as defined for group (a) above and R⁵ and R⁶ combine together to form a 5- to 7- membered carbocyclic or heterocyclic ring optionally substituted by groups as defined for R¹ above.

2. A compound according to claim 1 in which J is a group of formula (a).

3. A compound according to claims 1 or 2 in which R³ is hydrogen.

4. A compound according to any one of claims 1 to 3 wherein m is 0.

5. A compound according to any one of claims 1 to 4 wherein R² is methyl.

6. A compound according to any one of claims 1 to 5 wherein n is 0-2.

7. A compound according to any one of claims 1 to 6 wherein R⁴ is halogen, a C₁₋₆ alkyl group, a C₁₋₆ alkoxy group, benzyl, optionally substituted phenyl or a group (CH₂)_{q}NR⁸R⁹.

8. A compound according to claim 1 which is
7-(Indole-1-sulfonyl)-2,3,4,5-tetrahydro-1-*H*-benzo[d]azepine;
7-(4-Chloro-indole-1-sulfonyl))-2,3,4,5-tetrahydro-1-*H*-benzo[d] azepine;
7-(6-Chloro-indole-1-sulfonyl)-2,3,4,5-tetrahydro-1-*H*-benzo[d] azepine;
7-(3-Methyl-indole-1-sulfonyl)-2,3,4,5-tetrahydro-1-*H*-benzo[d] azepine;
7-[2-(4-Fluoro-phenyl)-indole-1-sulfonyl]-2,3,4,5-tetrahydro-1-*H*-benzo[d] azepine;
7-(2-Methyl-indole-1-sulfonyl)-2,3,4,5-tetrahydro-1-*H*-benzo[d] azepine;
Dimethyl-[1-(2,3,4,5-tetrahydro-1*H*-benzo[d]azepine-7-sulfonyl)-1*H*-indol-3-ylmethyl]-amine;
7-(3-Phenyl-indole-1-sulfonyl)-2,3,4,5-tetrahydro-1-*H*-benzo[d] azepine;
7-(3-Benzyl-indole-1-sulfonyl)-2,3,4,5-tetrahydro-1-*H*-benzo[d] azepine;
7-(7-Bromo-indole-1-sulfonyl)-2,3,4,5-tetrahydro-1-*H*-benzo[d] azepine;
7-(4-Methoxy-indole-1-sulfonyl)-2,3,4,5-tetrahydro-1-*H*-benzo[d] azepine;
7-(4-Methyl-indole-1-sulfonyl)-2,3,4,5-tetrahydro-1-*H*-benzo[d] azepine;
7-(5-Bromo-indole-1-sulfonyl)-2,3,4,5-tetrahydro-1-*H*-benzo[d] azepine;
7-(5-Fluoro-indole-1-sulfonyl)-2,3,4,5-tetrahydro-1-*H*-benzo[d] azepine;
7-(6-Methoxy-indole-1-sulfonyl)-2,3,4,5-tetrahydro-1-*H*-benzo[d] azepine;
7-(5-Chloro-2-methyl-indole-1-sulfonyl)-2,3,4,5-tetrahydro-1-*H*-benzo[d] azepine;
7-(6-Fluoro-indole-1-sulfonyl)-2,3,4,5-tetrahydro-1-*H-*benzo[d] azepine;
7-(7-Chloro-indole-1-sulfonyl)-2,3,4,5-tetrahydro-1-*H*-benzo[d] azepine;
9-(2,3,4,5-Tetrahydro-1*H*-benzo[d]azepine-7-sulfonyl)-2,3,4,9-tetrahydro-1H-carbazole;
7-(4,5,6,7-Tetrafluoro-indole-1-sulfonyl)-2,3,4,5-tetrahydro-1-*H* -benzo[d]azepine;
7-(2,3-Dimethyl-indole-1-sulfonyl)-2,3,4,5-tetrahydro-1-*H*-benzo[d] azepine;
7-(3-Chloro-indazole-1-sulfonyl)-2,3,4,5-tetrahydro-1-*H*-benzo[d] azepine;
7-(5,6-Dichloro-indole-1-sulfonyl)-2,3,4,5-tetrahydro-1-*H*-benzo[d] azepine;
7-(7-Nitro-indole-1-sulfonyl)-2,3,4,5-tetrahydro-1*H*-benzo[d]azepine;
9-(2,3,4,5-Tetrahydro-1*H*-benzo[d]azepine-7-sulfonyl)-9*H*-carbazole;
7-(4,6-Difluoro-indole-1-sulfonyl)-2,3,4,5-tetrahydro-1*H*-benzo[d]azepine;
7-(6-Trifluoromethyl-indole-1-sulfonyl)-2,3,4,5-tetrahydro-1*H* -benzo[d]azepine;
7-(5,6-Difluoro-mdole-1-sulfonyl)-2,3,4,5-tetrahydro-1*H*-benzo[d]azepine;
Dimethyl-[1-(2,3,4,5-tetrahydro-1*H*-benzo[d]azepine-7-sulfonyl)-1*H*-indol-3-ylmethyl]-amine;
7-(Indole-1-sulfonyl)-3-methyl-2,3,4,5-tetrahydro-1-*H*-benzo[d]azepine;
3-Ethyl-7-(indole-1-sulfonyl)-2,3,4,5-tetrahydro-1-*H*-benzo[d]azepine;
7-(2,3-Dihydro-indole-1-sulfonyl)-2,3,4,5-tetrahydro-1*H*-benzo[d]azepine;
7-(5-Bromo-2,3-dihydro-indole-1-sulfonyl)-2,3,4,5-tetrahydro-1*H*-benzo[d] azepine;
or a pharmaceutically acceptable salt thereof.

9. A process for the preparation of a compound of formula (I) or a pharmaceutically acceptable salt thereof, which process comprises the coupling of a compound of formula (II):
J-H (II)
in which J is as defined in formula (I) with a compound of formula (III) or a protected derivative thereof; 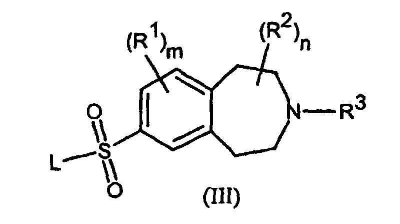 in which R¹, R², m and n are as defined in formula (I), R^{3a} is an N protecting group or R³ and L is a leaving group and optionally thereafter:
• converting a compound of formula (I) into another compound of formula (I);
• removing any protecting groups;
• forming a pharmaceutically acceptable salt.

10. A pharmaceutical composition which comprises a compound according to any one of claims 1 to 8 and a pharmaceutically acceptable carrier or excipient.

11. A compound according to any one of claims 1 to 8 for use in therapy.

12. A compound according to any one of claims 1 to 8 for use in the treatment of depression, anxiety, Alzheimers disease, age related cognitive decline, ADHD, obesity, mild cognitive impairment and schizophrenia.

13. The use of a compound of formula (I) as defined in any one of claims 1 to 8 or a pharmaceutically acceptable salt thereof in the manufacture of a medicament for the treatment or prophylaxis of depression, anxiety, Alzheimers disease, age related cognitive decline, ADHD, obesity, mild cognitive impairment and schizophrenia.

## Patentansprüche

1. Verbindung der Formel (I) oder ein pharmazeutisch verträgliches Salz davon: 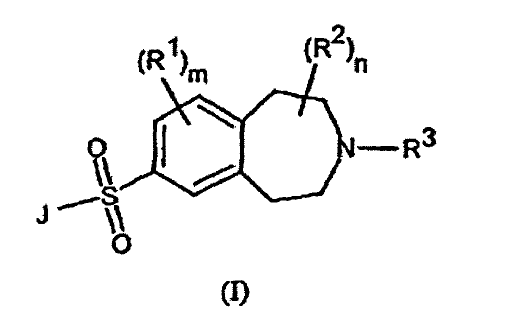 wobei
R¹ Halogen, C₁₋₆-Alkyl, C₁₋₆-Alkoxy, C₁₋₆-Alkanoyl, CN, CF₃ oder OCF₃ ist;
R² C₁₋₆-Alkyl ist;
R³ Wasserstoff oder ein C₁₋₁₀-Alkylrest, gegebenenfalls substituiert mit 1, 2 oder 3 Substituenten, ausgewählt aus Halogen, C₁₋₆-Alkoxy, CN, Amino, Mono- oder Di-C₁₋₆-alkylamino oder einem Rest -C(O)OR⁷, worin R⁷ Wasserstoff oder C₁₋₆-Alkyl ist, ist; m 0 bis 3 ist; n 0 bis 8 ist;
J ausgewählt ist aus einem Rest der Formel (a), (b) oder (c), wobei
(a) ein Rest 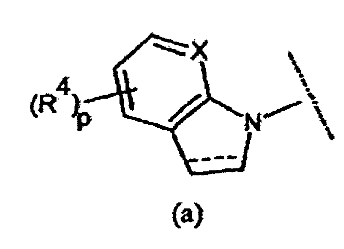 ist,
wobei R⁴ Halogen, C₁₋₆-Alkyl, C₁₋₆-Alkoxy, C₁₋₆-Alkanoyl, C₁₋₆-Alkylthio, Hydroxy, CN, CF₃, NO₂, OCF₃, Phenyl, gegebenenfalls substituiert mit Resten wie oben für R¹ definiert, Benzyl, Phenyloxy, Benzyloxy oder C₃₋₆-Cycloalkyloxy oder ein Rest (CH₂)_{q}NR⁸R⁹ ist, worin q 0, 1 oder 2 ist und R⁸ und R⁹ unabhängig Wasserstoff oder C₁₋₆-Alkyl sind;
p 0, 1, 2, 3 oder 4 ist;
X CH oder N ist; und
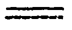 eine Einfach- oder Doppelbindung ist;
(b) ein Rest 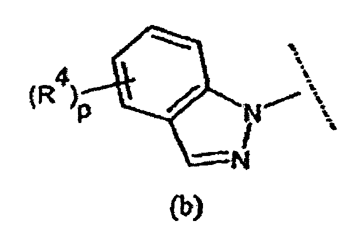 ist,
worin R⁴ und p wie oben für Rest (a) definiert sind; und
(c) ein Rest 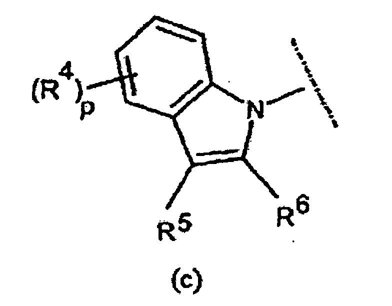 ist,
worin R⁴ und p wie oben für Rest (a) definiert sind und R⁵ und R⁶ miteinander einen 5- bis 7-gliedrigen carbocyclischen oder heterocyclischen Ring, gegebenenfalls substituiert mit Resten, wie oben für R¹ definiert, bilden.

2. Verbindung nach Anspruch 1, wobei J ein Rest der Formel (a) ist.

3. Verbindung nach den Ansprüchen 1 oder 2, wobei R³ Wasserstoff ist.

4. Verbindung nach einem der Ansprüche 1 bis 3, wobei m 0 ist.

5. Verbindung nach einem der Ansprüche 1 bis 4, wobei R² Methyl ist.

6. Verbindung nach einem der Ansprüche 1 bis 5, wobei n 0 bis 2 ist.

7. Verbindung nach einem der Ansprüche 1 bis 6, wobei R⁴ Halogen, ein C₁₋₆-Alkylrest, ein C₁₋₆-Alkoxyrest, Benzyl, gegebenenfalls substituiertes Phenyl oder ein Rest (CH₂)_{q}NR⁸R⁹ ist.

8. Verbindung nach Anspruch 1, nämlich:
7-(Indol-1-sulfonyl)-2,3,4,5-tetrahydro-1-*H*-benzo[d]azepin;
7-(4-Chlorindol-1-sulfonyl)-2,3,4,5-tetrahydro-1-*H*-benzo[d]azepin;
7-(6-Chlorindol-1-sulfonyl)-2,3,4,5-tetrahydro-1-*H*-benzo[d]azepin;
7-(3-Methylindol-1-sulfonyl)-2,3,4,5-tetrahydro-1-*H*-benzo[d]azepin;
7-[2-(4-Fluorphenyl)-indol-1-sulfonyl]-2,3,4,5-tetrahydro-1-*H*-benzo[d]azepin;
7-(2-Methylindol-1-sulfonyl)-2,3,4,5-tetrahydro-1-*H*-benzo[d]azepin;
Dimethyl-[1-(2,3,4,5-tetrahydro-1*H*-benzo[d]azepin-7-sulfonyl)-1*H*-indol-3-ylmethyl]-amin;
7-(3-Phenylindol-1-sulfonyl)-2,3,4,5-tetrahydro-1-*H*-benzo[d]azepin;
7-(3-Benzylindol-1-sulfonyl)-2,3,4,5-tetrahydro-1-*H*-benzo[d]azepin;
7-(7-Bromindol-1-sulfonyl)-2,3,4,5-tetrahydro-1-*H*-benzo[d]azepin;
7-(4-Methoxyindol-1-sulfonyl)-2,3,4,5-tetrahydro-1-*H*-benzo[d]azepin;
7-(4-Methylindol-1-sulfonyl)-2,3,4,5-tetrahydro-1-*H*-benzo[d]azepin;
7-(5-Bromindol-1-sulfonyl)-2,3,4,5-tetrahydro-1-*H*-benzo[d]azepin;
7-(5-Fluorindol-1-sulfonyl)-2,3,4,5-tetrahydro-1-*H*-benzo[d]azepin;
7-(6-Methoxyindol-1-sulfonyl)-2,3,4,5-tetrahydro-1-*H*-benzo[d]azepin;
7-(5-Chlor-2-methylindol-1-sulfonyl)-2,3,4,5-tetrahydro-1-*H*-benzo[d]azepin;
7-(6-Fluorindol-1-sulfonyl)-2,3,4,5-tetrahydro-1-*H*-benzo[d]azepin;
7-(7-Chlorindol-1-sulfonyl)-2,3,4,5-tetrahydro-1-*H*-benzo[d]azepin;
9-(2,3,4,5-Tetrahydro-1*H*-benzo[d]azepin-7-sulfonyl)-2,3,4,9-tetrahydro-1*H*-carbazol;
7-(4,5,6,7-Tetrafluorindol-1-sulfonyl)-2,3,4,5-tetrahydro-1-*H*-benzo[d]azepin;
7-(2,3-Dimethylindol-1-sulfonyl)-2,3,4,5-tetrahydro-1-*H*-benzo[d]azepin;
7-(3-Chlorindazol-1-sulfonyl)-2,3,4,5-tetrahydro-1-*H*-benzo[d]azepin;
7-(5,6-Dichlorindol-1-sulfonyl)-2,3,4,5-tetrahydro-1-*H*-benzo[d]azepin;
7-(7-Nitroindol-1-sulfonyl)-2,3,4,5-tetrahydro-1*H*-benzo[d]azepin;
9-(2,3,4,5-Tetrahydro-1*H*-benzo[d]azepin-7-sulfonyl)-9*H*-carbazol;
7-(4,6-Difluorindol-1-sulfonyl)-2,3,4,5-tetrahydro-1*H*-benzo[d]azepin;
7-(6-Trifluormethylindol-1-sulfonyl)-2,3,4,5-tetrahydro-1*H*-benzo[d]azepin;
7-(5,6-Difluorindol-1-sulfonyl)-2,3,4,5-tetrahydro-1*H*-benzo[d]azepin;
Dimethyl-[1-(2,3,4,5-tetrahydro-1*H*-benzo[d]azepin-7-sulfonyl)-1*H*-indol-3-ylmethyl]-amin;
7-(Indol-1-sulfonyl)-3-methyl-2,3,4,5-tetrahydro-1-*H*-benzo[d]azepin;
3-Ethyl-7-(indol-1-sulfonyl)-2,3,4,5-tetrahydro-1-*H*-benzo[d]azepin;
7-(2,3-Dihydroindol-1-sulfonyl)-2,3,4,5-tetrahydro-1*H*-benzo[d]azepin;
7-(5-Brom-2,3-dihydroindol-1-sulfonyl)-2,3,4,5-tetrahydro-1*H*-benzo[d]azepin;
oder ein pharmazeutisch verträgliches Salz davon.

9. Verfahren zur Herstellung einer Verbindung der Formel (I) oder eines pharmazeutisch verträglichen Salzes davon, wobei das Verfahren das Koppeln einer Verbindung der Formel (II):
J-H (II)
worin J wie in Formel (I) definiert ist, mit einer Verbindung der Formel (III) oder einem geschützten Derivat davon; 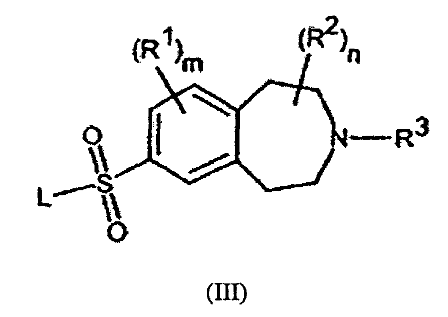 worin R¹, R², m und n wie in Formel (I) definiert sind, R^{3a} eine N-Schutzgruppe oder R³ ist und L eine Abgangsgruppe ist, und gegebenenfalls darauffolgend:
• Umwandeln einer Verbindung der Formel (I) in eine andere Verbindung der Formel (I);
• Entfernen aller Schutzgruppen;
• Bilden eines pharmazeutisch verträglichen Salzes
umfasst.

10. Arzneimittel, umfassend eine Verbindung nach einem der Ansprüche 1 bis 8 und einen pharmazeutisch verträglichen Träger oder Exzipienten.

11. Verbindung nach einem der Ansprüche 1 bis 8 zur Verwendung in der Therapie.

12. Verbindung nach einem der Ansprüche 1 bis 8 zur Verwendung bei der Behandlung von Depression, Angst, Alzheimer-Krankheit, altersbedingtem kognitiven Verfall, ADHD, Obesität, leichter kognitiver Beeinträchtigung und Schizophrenie.

13. Verwendung einer Verbindung der Formel (I) wie in einem der Ansprüche 1 bis 8 definiert oder eines pharmazeutisch verträglichen Salzes davon bei der Herstellung eines Medikaments zur Behandlung oder Prophylaxe von Depression, Angst, Alzheimer-Krankheit, altersbedingtem kognitiven Verfall, ADHD, Obesität, leichter kognitiver Beeinträchtigung und Schizophrenie.

## Revendications

1. Composé de formule (I) ou un de sels pharmaceutiquement acceptables : 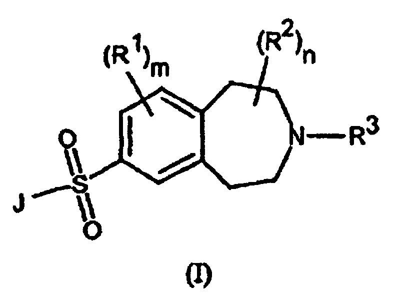 formule dans laquelle
R¹ représente un atome d'halogène, un groupe alkyle en C₁ à C₆, alkoxy en C₁ à C₆, alcanoyle en C₁ à C₆, CN, CF₃ ou OCF₃ ;
R² représente un groupe alkyle en C₁ à C₆ ;
R³ représente un atome d'hydrogène ou un groupe alkyle en C₁ à C₁₀ facultativement substitué avec un, deux ou trois substituants choisis dans le groupe consistant en des substituants halogéno, alkoxy en C₁ à C₆, CN, amino, mono- ou di (alkyle en C₁ à C₆) amino ou un groupe -C(O)OR⁷ dans lequel R⁷ représente un atome d'hydrogène ou un groupe alkyle en C₁ à C₆ ; m a une valeur de 0 à 3 ;
n a une valeur de 0 à 8 ;
J est choisi dans un groupe de formules (a), (b) et (c), dans lequel
(a) représente un groupe 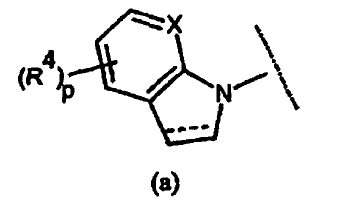 dans lequel R⁴ représente un atome d'halogène, un groupe alkyle en C₁ à C₆, alkoxy en C₁ à C₆, alcanoyle en C₁ à C₆, alkylthio en C₁ à C₆, hydroxy, CN, CF₃, NO₂, OCF₃, phényle facultativement substitué avec des groupes tels que définis pour R¹ ci-dessus, benzyle, phényloxy, benzyloxy ou cycloalkyloxy en C₃ à C₆ ou un groupe (CH₂)_{q}NR⁸R⁹ dans lequel q est égal à 0, 1 ou 2 et R⁸ et R⁹ représentent indépendamment un atome d'hydrogène ou un groupe alkyle en C₁ à C₆ ; 1
p est égal 0, 1, 2, 3 ou 4 ;
X représente un groupe CH ou N ; et
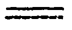 représente une liaison simple ou une double liaison ;
(b) représente un groupe 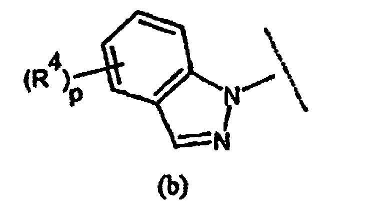 dans lequel R⁴ et p répondent aux définitions mentionnées pour le groupe (a) ci-dessus ; et
(c) représente un groupe 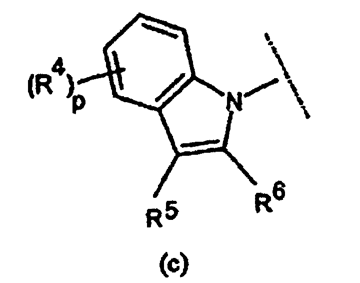
dans lequel R⁴ et p répondent aux définitions mentionnées pour le groupe (a) ci-dessus et R⁵ et R⁶ se combinent pour former un noyau carbocyclique ou hétérocyclique penta- à heptagonal facultativement substitué avec des groupes tels que définis pour R¹ ci-dessus.

2. Composé suivant la revendication 1, dans lequel J représente un groupe de formule (a).

3. Composé suivant la revendication 1 ou 2, dans lequel R³ représente un atome d'hydrogène.

4. Composé suivant l'une quelconque des revendications 1 à 3, dans lequel m est égal à 0.

5. Composé suivant l'une quelconque des revendications 1 à 4, dans lequel R² représente un groupe méthyle.

6. Composé suivant l'une quelconque des revendications 1 à 5, dans lequel n a une valeur de 0 à 2.

7. Composé suivant l'une quelconque des revendications 1 à 6, dans lequel R⁴ représente un atome d'halogène, un groupe alkyle en C₁ à C₆, un groupe alkoxy en C₁ à C₆, un groupe benzyle, un groupe phényle facultativement substitué ou un groupe (CH₂)_{q}NR⁸R⁹.

8. Composé suivant la revendication 1, qui est
la 7-(indole-1-sulfonyl)-2,3,4,5-tétrahydro-1-*H*-benzo[d]azépine ;
la 7-(4-chloro-indole-1-sulfonyl)-2,3,4,5-tétrahydro-1-*H*-benzo[d]azépine ;
la 7-(6-chloro-indole-1-sulfonyl)-2,3,4,5-tétrahydro-1-*H*-benzo[d]azépine ;
la 7-(3-méthylindole-2-sulfonyl)-2,3,4,5-tétrahydro-1-*H*-benzo[d]azépine ;
la 7-[2-(4-fluorophényl)-indole-1-sulfonyl]-2,3,4,5-tétrahydro-1-*H*-benzo[d]azépine ;
la 7-(2-méthylindole-1-sulfonyl)-2,3,4,5-tétrahydro-1-*H*-benzo[d]azépine ;
la diméthyl-[1-(2,3,4,5-tétrahydro-1*H*-benzo[d]azépine-7-sulfonyl)-1*H*-indol-3-ylméthyl]-amine ;
la 7-(3-phénylindole-1-sulfonyl)-2,3,4,5-tétrahydro-1-H-benzo[d]azépine ;
la 7-(3-benzylindole-1-sulfonyl)-2,3,4,5-tétrahydro-1-*H*-benzo[d]azépine ;
la 7-(7-bromo-indole-1-sulfonyl)-2,3,4,5-tétrahydro-1-*H*-benzo[d]azépine ;
la 7-(4-méthoxyindole-1-sulfonyl)-2,3,4,5-tétrahydro-1-*H*-benzo[d]azépine ;
la 7-(4-méthylindole-1-sulfonyl)-2,3,4,5-tétrahydro-1-*H*-benzo[d]azépine ;
la 7-(5-bromo-indole-1-sulfonyl)-2,3,4,5-tétrahydro-1-*H*-benzo[d]azépine ;
la 7-(5-fluoro-indole-1-sulfonyl)-2,3,4,5-tétrahydro-1-*H*-benzo[d]azépine ;
la 7-(6-méthoxyindole-1-sulfonyl)-2,3,4,5-tétrahydro-1-*H*-benzo[d]azépine ;
la 7-(5-chloro-2-méthylindole-1-sulfonyl)-2,3,4,5-tétrahydro-1-*H*-benzo[d]azépine ;
la 7-(6-fluoro-indole-1-sulfonyl)-2,3,4,5-tétrahydro-1-*H*-benzo[d]azépine ;
la 7-(7-chloro-indole-1-sulfonyl)-2,3,4,5-tétrahydro-1-*H*-benzo[d]azépine ;
le 9-(2,3,4,5-tétrahydro-1H-benzo[d]azépine-7-sulfonyl)-2,3,4,9-tétrahydro-1H-carbazole ;
la 7-(4,5,6,7-tétrafluoro-indole-1-sulfonyl)-2,3,4,5-tétrahydro-1-*H*-benzo[d]azépine ;
la 7-(2,3-diméthylindole-1-sulfonyl)-2,3,4,5-tétrahydro-1-*H*-benzo[d]azépine ;
la 7-(3-chloro-indazole-1-sulfonyl)-2,3,4,5-tétrahydro-1-*H*-benzo[d]azépine ;
la 7-(5,6-dichloro-indole-1-sulfonyl)-2,3,4,5-tétrahydro-1-*H*-benzo[d]azépine ;
la 7-(7-nitro-indole-1-sulfonyl)-2,3,4,5-tétrahydro-1-*H*-benzo[d]azépine ;
le 9-(2,3,4,5-tétrahydro-1*H*-benzo[d]azépine-7-sulfonyl)-9*H*-carbazole ;
la 7-(4,6-difluoro-indole-1-sulfonyl)-2,3,4,5-tétrahydro-1-*H*-benzo[d]azépine ;
la 7-(6-trifluorméthylindole-1-sulfonyl)-2,3,4,5-tétrahydro-1-*H*-benzo[d]azépine ;
la 7-(5,6-difluoro-indole-1-sulfonyl)-2,3,4,5-tétrahydro-1-*H*-benzo[d]azépine ;
la diméthyl-[1-(2,3,4,5-tétrahydro-1*H*-benzo[d]azépine-7-sulfonyl)-1*H*-indol-3-ylméthyl]-amine ;
la 7-(indole-1-sulfonyl)-3-méthyl-2,3,4,5-tétrahydro-1-*H*-benzo[d]azépine ;
la 3-éthyl-7-(indole-1-sulfonyl)-2,3,4,5-tétrahydro-1-*H*-benzo[d]azépine ;
la 7-(2,3-dihydro-indole-1-sulfonyl)-2,3,4,5-tétrahydro-1-*H*-benzo[d]azépine ;
la 7-(5-bromo-2,3-dihydro-indole-1-sulfonyl)-2,3,4,5-tétrahydro-1-*H*-benzo[d]azépine ;
ou un de ses sels pharmaceutiquement acceptables.

9. Procédé pour la préparation d'un composé de formule (I) ou d'un de ses sels pharmaceutiquement acceptables, procédé qui comprend le couplage d'un composé de formule (II) :
**J - H** (II)
dans laquelle J répond à la définition mentionnée pour la formule (I) avec un composé de formule (III) ou un de ses dérivés protégés ; 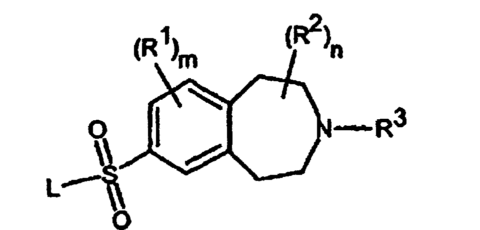 formule dans laquelle R¹, R², m et n répondent aux définitions mentionnées pour la formule (I), R^{3a} représente un groupe protecteur de l'atome de N ou bien R³ et L représentent un groupe partant, et ensuite, facultativement :
• la conversion d'un composé de formule (I) en un autre composé de formule (I) ;
• l'élimination de tous les groupes protecteurs ;
• la formation d'un sel pharmaceutiquement acceptable.

10. Composition pharmaceutique qui comprend un composé suivant l'une quelconque des revendications 1 à 8 ou un support ou excipient pharmaceutiquement acceptable.

11. Composé suivant l'une quelconque des revendications 1 à 8, destiné à être utilisé en thérapeutique.

12. Composé suivant l'une quelconque des revendications 1 à 8, destiné à être utilisé dans le traitement de la dépression, de l'anxiété, de la maladie d'Alzheimer, du déclin cognitif dû à l'âge, du syndrome de déficit d'attention/hyperactivité (ADHD), de l'obésité, de l'altération cognitive faible et de la schizophrénie.

13. Utilisation d'un composé de formule (I) tel que défini dans l'une quelconque des revendications 1 à 8 ou d'un de ses sels pharmaceutiquement acceptables dans la production d'un médicament destiné au traitement ou à la prophylaxie de la dépression, de l'anxiété, de la maladie d'Alzheimer, du déclin cognitif dû à l'âge, du syndrome de déficit d'attention/hyperactivité (ADHD), de l'obésité, d'une altération cognitive faible et de la schizophrénie.
